Europäisches Patentamt

**European Patent Office**

Office européen des brevets

(19)

(11) Publication number: **0 232 107**
**A2**

(12) # EUROPEAN PATENT APPLICATION

(21) Application number: **87300674.6**

(22) Date of filing: **27.01.87**

(51) Int. Cl.⁴: **C 12 N 15/00**
C 12 P 21/02, C 12 N 1/20
//(C12N1/20,C12R1:19)

(30) Priority: **05.02.86 JP 23319/86**

(43) Date of publication of application:
**12.08.87 Bulletin 87/33**

(84) Designated Contracting States:
**AT BE CH DE ES FR GB GR IT LI LU NL SE**

(71) Applicant: **KYOWA HAKKO KOGYO CO., LTD.**
**6-1, Ohte-Machi Itchome**
**Chiyoda-ku Tokyo (JP)**

(72) Inventor: **Miyaji, Hiromasa c/o Patent Department**
**Kyowa Hakko Kogyo Co. Ltd. 6-1 Ohtemachi Itchome**
**Chiyoda-ku Tokyo (JP)**

**Nishi, Tatsunari c/o Patent Department**
**Kyowa Hakko Kogyo Co. Ltd. 6-1 Ohtemachi Itchome**
**Chiyoda-ku Tokyo (JP)**

**Itoh, Seiga c/o Patent Department**
**Kyowa Hakko Kogyo Co. Ltd. 6-1 Ohtemachi Itchome**
**Chiyoda-ku Tokyo (JP)**

(74) Representative: **Lambert, Hugh Richmond et al**
**D. YOUNG & CO. 10 Staple Inn**
**London, WC1V 7RD (GB)**

The applicant has filed a statement in accordance with Rule 28 (4) EPC (issue of a sample only to an expert). Accession numbers of the deposits: Ferm. B.P. 981 - Ferm. B.P. 963.

(54) **Human lymphotoxin polypeptide derivative.**

(57) A modified human lymphotoxin polypeptide derivative (HLPD) is disclosed having high LT activity. The modified polypeptide derivative corresponds to the normal human lymphotoxin polypeptide, the amino acid sequence for which is given, the modification being that certain of the amino acids are missing, particularly one or more of the first twenty-two amino acids from the N-terminal end of the sequence. The modified HLPD is produced by recombinant DNA technology, and the corresponding DNA sequences are disclosed as well as recombinant plasmids and microorganisms transformed therewith and capable of expressing the modified HLPD in high yields and at low cost.

EP 0 232 107 A2

**Description**

## HUMAN LYMPHOTOXIN POLYPEPTIDE DERIVATIVE

The present invention relates to a novel human lymphotoxin polypeptide derivative, a DNA fragment coding for the polypeptide derivative, a recombinant plasmid wherein the DNA fragment is incorporated, a microorganism containing the plasmid, and a process for producing the human lymphotoxin polypeptide derivative using the microorganism.

Lymphotoxin (hereinafter referred to as LT) is a lymphokine produced by activated lymphocytes. It has been reported that LT shows a strong cytotoxic activity on some cancer cells and does not act on normal cells, and its clinical application as a carcinostatic substance has been expected. The human lymphotoxin polypeptide derivative (hereinafter referred to as HLTD) of the present invention also has an LT activity and its utilization as a medicament can be expected as well as LT.

Recombinant DNA technology has now been developed to the extent that the mass production of substances which are produced only in a small amount in cells of higher animals and are hard to isolate has become possible by using microorganisms.

As for LT, it has been reported by Gray, et al. that cDNA derived from human peripheral lymphocytes was cloned into Escherichia coli and its nucleotide sequence was determined [Patrick W. Gray, et al.: Nature 312, 721 (1984)].

In accordance with the present invention a method is provided for producing a large amount of a polypeptide having a high LT activity, at a low cost.

For this purpose, LT cDNA derived from LukII human lymphoblastoid cell line [Berish Y. Rubin, et al., Proc. Natl. Acad. Sci., USA 82, 6637 (1985)] has been cloned into Escherichia coli and the complete nucleotide sequence of the cDNA has been determined. The nucleotide sequence, and the coded amino acid sequence, is shown in Formula 1. The coded amino acid sequence is identical with the one reported by Gray, et al.

Following that, the present applicants have found that HLTD having a high LT activity can be produced at low cost by modifying the LT cDNA shown in Formula 1, cloning the modified cDNA into Escherichia coli and culturing the transformed Escherichia coli, thereby to express the HLTD product, and recovering the product HLTD.

## Formula 1

```
          10        20        30        40        50        60
ATGACACCACCTGAACGTCTCTTCCTCCCAAGGGTGTGTGGCACCACCCTACACCTCCTC
MetThrProProGluArgLeuPheLeuProArgValCysGlyThrThrLeuHisLeuLeu
-34

          70        80        90       100       110       120
CTTCTGGGGCTGCTGCTGGTTCTGCTGCCTGGGGCCCAGGGGCTCCCTGGTGTTGGCCTC
LeuLeuGlyLeuLeuLeuValLeuLeuProGlyAlaGlnGlyLeuProGlyValGlyLeu
                                                    1

         130       140       150       160       170       180
ACACCTTCAGCTGCCCAGACTGCCCGTCAGCACCCCAAGATGCATCTTGCCCACAGCACC
ThrProSerAlaAlaGlnThrAlaArgGlnHisProLysMetHisLeuAlaHisSerThr

         190       200       210       220       230       240
CTCAAACCTGCTGCTCACCTCATTGGAGACCCCAGCAAGCAGAACTCACTGCTCTGGAGA
LeuLysProAlaAlaHisLeuIleGlyAspProSerLysGlnAsnSerLeuLeuTrpArg

         250       260       270       280       290       300
GCAAACACGGACCGTGCCTTCCTCCAGGATGGTTTCTCCTTGAGCAACAATTCTCTCCTG
AlaAsnThrAspArgAlaPheLeuGlnAspGlyPheSerLeuSerAsnAsnSerLeuLeu

         310       320       330       340       350       360
GTCCCCACCAGTGGCATCTACTTCGTCTACTCCCAGGTGGTCTTCTCTGGGAAAGCCTAC
ValProThrSerGlyIleTyrPheValTyrSerGlnValValPheSerGlyLysAlaTyr

         370       380       390       400       410       420
TCTCCCAAGGCCACCTCCTCCCCACTCTACCTGGCCCATGAGGTCCAGCTCTTCTCCTCC
SerProLysAlaThrSerSerProLeuTyrLeuAlaHisGluValGlnLeuPheSerSer

         430       440       450       460       470       480
CAGTACCCCTTCCATGTGCCTCTCCTCAGCTCCCAGAAGATGGTGTATCCAGGGCTGCAG
GlnTyrProPheHisValProLeuLeuSerSerGlnLysMetValTyrProGlyLeuGln

         490       500       510       520       530       540
GAACCCTGGCTGCACTCGATGTACCACGGGGCTGCGTTCCAGCTCACCCAGGGAGACCAG
GluProTrpLeuHisSerMetTyrHisGlyAlaAlaPheGlnLeuThrGlnGlyAspGln

         550       560       570       580       590       600
CTATCCACCCACACAGATGGCATCCCCCACCTAGTCCTCAGCCCTAGTACTGTCTTCTTT
LeuSerThrHisThrAspGlyIleProHisLeuValLeuSerProSerThrValPhePhe

         610       620
GGAGCCTTCGCTCTGTAG
GlyAlaPheAlaLeu***
        171
```

In more detail, the present invention provides a novel HLTD, a DNA fragment coding for the HLTD, a recombinant plasmid wherein the DNA fragment is incorporated, a microorganism containing the plasmid, and a process for producing the novel HLTD using the microorganism.

The HLTD of the present invention is a polypeptide wherein certain amino acids are removed from a mature human LT polypeptide having the amino acid sequence from Leu (No. 1) to Leu (No. 171) shown in Formula 1. The amino acids to be removed are those near the N-terminal, and preferably at least one of the first to 22nd amino acids from the N-terminal.

The recombinant plasmid of the present invention is a plasmid wherein a DNA fragment coding for the HLTD is incorporated into an appropriate plasmid having a function to express the DNA.

The DNA fragment used in the present invention codes for the HLTD that preferably has the amino acid sequence wherein at least one of the first to 22nd amino acids are removed from the amino acid sequence of the mature human LT polypeptide shown in Formula 1. As the DNA coding for the human LT shown in Formula 1, cDNA (human LT cDNA) obtainable by reverse transcription from a messenger RNA coding for LT by recombinant DNA technology, a DNA coding for LT obtainable from chromosomal DNA, etc. can be utilized.

As the human LT cDNA, any cDNA can be used so far as it codes for human LT. Specifically, pLT1 or λLT11 can be used. pLT1 was prepared by the present inventors, and the process for preparation is disclosed in Reference Example 1. LT DNA in pLT1 has the nucleotide sequence shown in Formula 1, which was determined by dideoxy chain termination method using M13 phage [J. Messing, et al.: Gene 19, 269 (1985)]. λLT11 was disclosed in Nature 312, 721 - 724 (1984).

Removal of the nucleotide sequence from DNA is carried out by cleavage with restriction enzymes and digestion with nuclease BAL31 or DNA polymerase I•Klenow fragment (hereinafter referred to as "Klenow fragment").

As the plasmid to incorporate a DNA coding for HLTD, any plasmid can be used so long as the DNA incorporated therein can be expressed in Escherichia coli. Preferably, a plasmid wherein a foreign DNA can be inserted downstream from a suitable promoter such as trp promoter or lac promoter and the length between Shine-Dalgarno sequence (referred to as SD sequence hereinafter) and initiation codon (ATG) is adjusted, for example, to 6 - 18 base pairs is employed. Preferred examples are pKYP10, pKYP11 and pKYP12 which were constructed by the present inventors (Japanese Published Unexamined Patent Application No. 110600/83). To give an initiation codon to the DNA coding for HLTD, a plasmid (ATG vector) which has the initiation codon downstream from the promoter and retains the initiation codon at the 3'terminal side when cleaved with a restriction enzyme is used. Preferred examples thereof are pTrS3 (Japanese Published Unexamined Patent Application Nos. 67297/84 and 140883/84), and pTrS20 and pTrS10 shown in Reference Examples 2 and 3, respectively.

An example of a construction of a recombinant plasmid having an incorporated DNA coding for HLTD is described below and illustrated by the accompanying drawings, wherein pLT1 is used as the human LT cDNA, pKYP10 as the plasmid for incorporating the cDNA, and pTrS20 and pTrS10 as the plasmids for giving the initiation codon. In the drawings:

Fig. 1 is a flow sheet showing the steps for constructing plasmid pLA1. As for HaeIII, only the site employed for the construction is shown.

Fig. 2 is a flow sheet showing the steps for constructing plasmid pLSA1.

Fig. 3 is a flow sheet showing the steps for constructing plasmid pLC2.

Fig. 4 is a flow sheet showing the steps for constructing plasmids pLdTD11 and pLdTD12.

Fig. 5 is a flow sheet showing the steps for constructing plasmid pLdTA2.

Fig. 6 (1) and (2) show the outline of the process for synthesizing cDNA and for constructing a recombinant plasmid containing the DNA according to the Okayama-Berg method.

Fig. 7 shows the complete nucleotide sequence of the cDNA coding for human LT contained in pLT1.

Fig. 8 is a flow sheet showing the steps for constructing ATG vector pTrS20.

Fig. 9 is a flow sheet showing the steps for constructing ATG vector pTrS10.

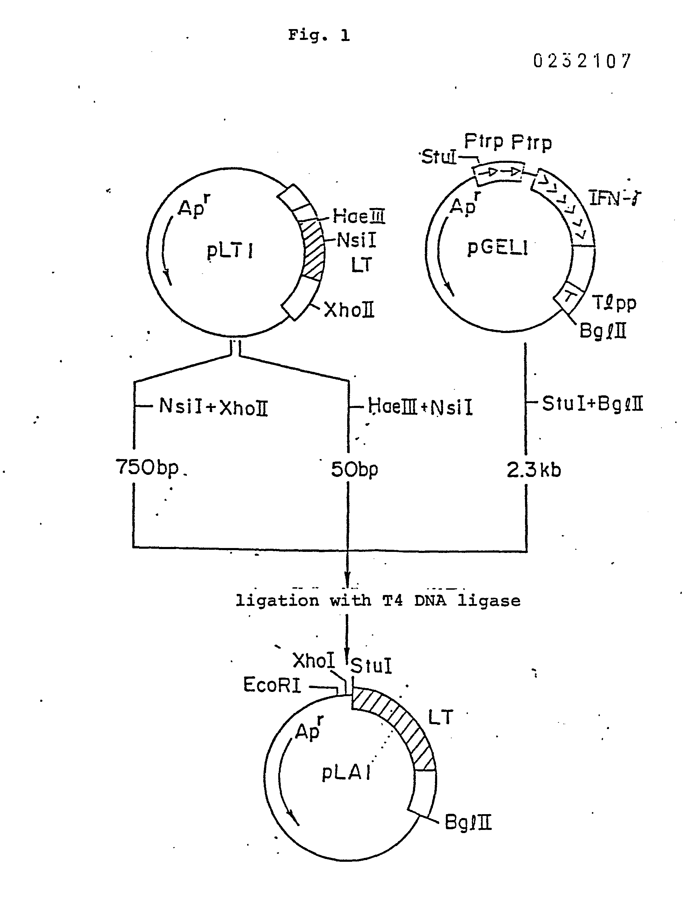

As illustrated in Fig. 1, pLT1 is cleaved with NsiI and XhoII and a DNA fragment of about 750 base pairs (a base pair will be hereinafter referred to as "bp") is purified by low-gelling-temperature agarose gel electrophoresis (LGT method) [L. Wieslander: Analytical Biochemistry 98, 305 (1979)]. pLT1 is also cleaved with HaeIII and NsiI, and a DNA fragment of about 50 bp is purified by polyacrylamide gel electrophoresis [A. M. Maxam, et al.: Proc. Natl. Acad. Sci., USA 74, 560 (1977)]. Separately, pGEL1 [Sekine, et al.: Proc. Natl. Acad. Sci., USA 82, 4306 (1985)] is cleaved with StuI and Bg1II, and a DNA fragment of about 2.3 kilobases (a kilobase will be hereinafter referred to as "Kb") is purified. The thus obtained DNA fragments are ligated with T4 DNA ligase to obtain pLA1.

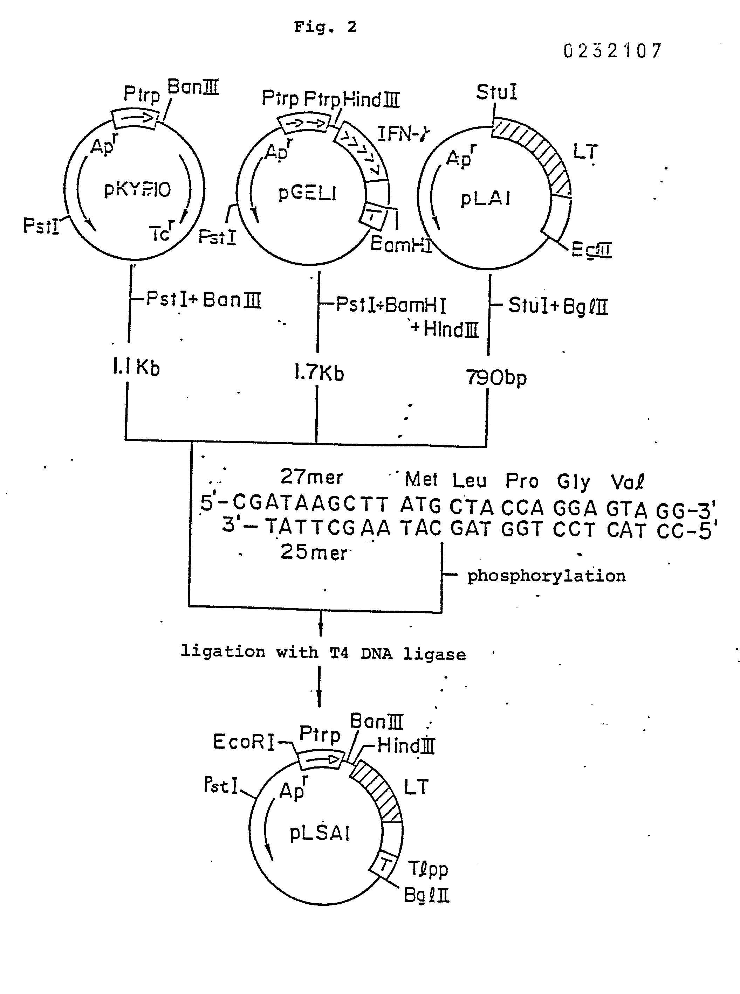

Then, as illustrated in Fig. 2 pLA1 is cleaved with StuI and Bg1II, and a DNA fragment of about 790 bp is purified. pKYP10 is cleaved with BanIII and PstI, and a DNA fragment of about 1.1 Kb is purified. pGEL1 is also cleaved with PstI, BamHI and HindIII, and a DNA fragment of about 1.7 Kb is purified. The thus obtained DNA fragments and a synthetic DNA shown in Fig. 2, which contains an initiation codon (ATG) and a sequence from the triplet (CTA) coding for leucin (Leu) as the first amino acid of the mature LT polypeptide to the second base (GG) of the triplet (GGC) coding for glycine (Gly) as the fifth amino acid, are ligated with T4 DNA ligase to obtain pLSA1.

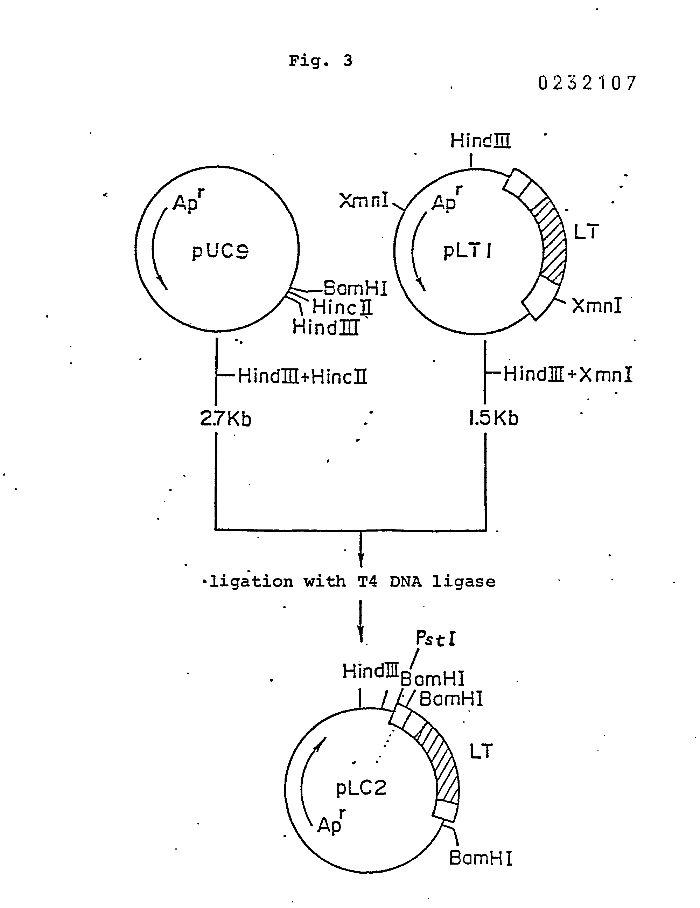

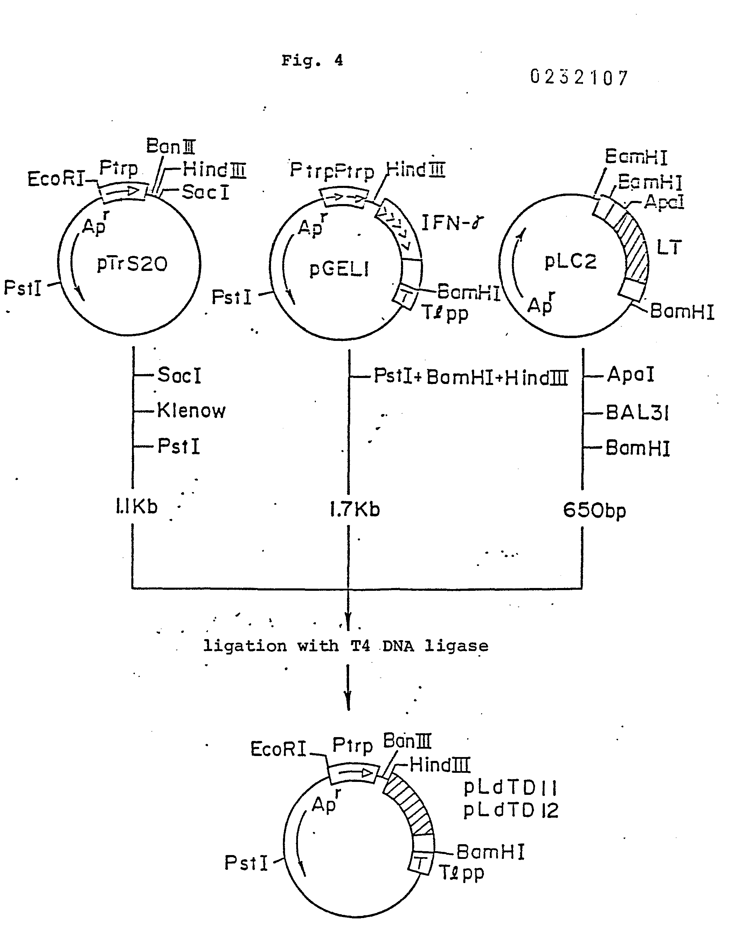

To obtain a DNA coding for HLTD wherein the N-terminal is deleted, pLT1 is cleaved with HindIII and XmnI as illustrated in Fig. 3, and a DNA fragment of about 1.5 Kb is purified. pUC9 (Jeffrey Vieira, et al.: Gene 19, 259 (1982)] is cleaved with HindIII and HincII, and a DNA fragment of about 2.7 Kb is purified. The thus obtained fragments are ligated with T4 DNA ligase to obtain pLC2. Then, as illustrated in Fig. 4, pLC2 is cleaved with Apal, acted on by BAL31 for 10 seconds to 30 minutes to cut out a DNA coding for the N-terminal amino acids of LT, and cleaved with BamHI, and a DNA fragment of about 650 bp is purified. Separately, a vector pTrS20 containing an initiation codon is digested with SacI, treated with Klenow fragment, and further cleaved with PstI, and a DNA fragment of about 1.1 Kb is purified. pGEL1 is also cleaved with BamHI, PstI and HindIII, and a DNA fragment of about 1.7 Kb is purified. The thus obtained DNA fragments are ligated with T4 DNA ligase to obtain a recombinant plasmid wherein the DNA coding for HLTD is incorporated. The plasmids obtained in Examples were named pLdTD11 and pLdTD12.

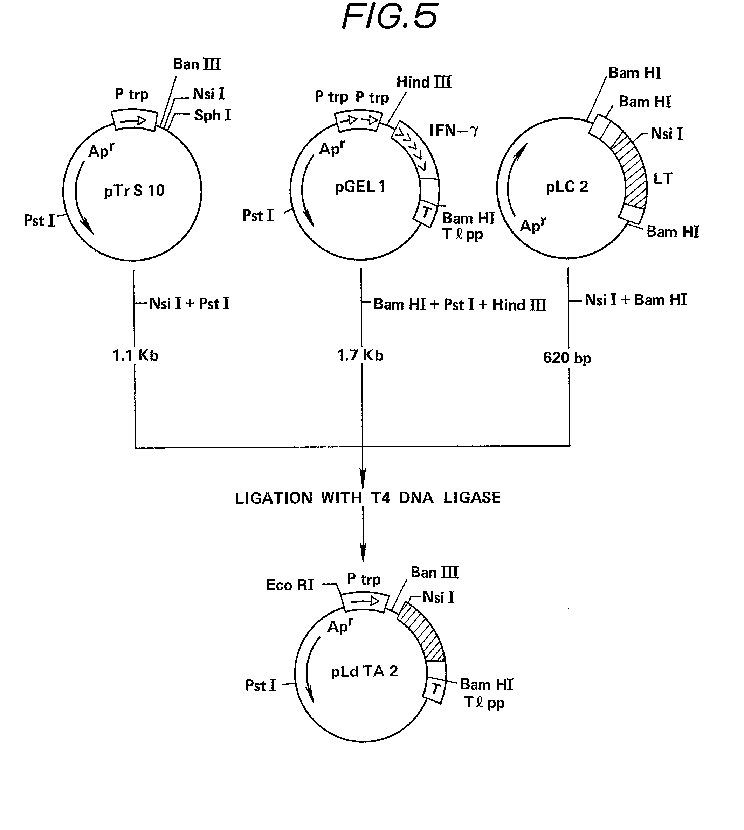

A process for preparing plasmid pLdTA2 coding for an LT derivative wherein the sequence from Leu as the N-terminal amino acid of the mature human LT to the 19th amino acid lysine (Lys) thereof is deleted and the 20th amino acid Met is the N-terminal amino acid is illustrated in Fig. 5. pLC2 is cleaved with BamHI and NsiI, and a DNA fragment of about 620 bp is purified. pTrS10 is cleaved with NsiI and PstI, and a DNA fragment of about 1.1 Kb is purified. Further, pGEL1 is cleaved with BamHI, PstI and HindIII, and a DNA fragment of about 1.7 Kb is purified. The thus obtained DNA fragments are ligated with T4 DNA ligase to obtain pLdTA2.

Reaction conditions required for the recombinant DNA technology described above are generally as follows.

Digestion of DNA with restriction enzymes is usually carried out by reacting 0.1 to 20 µg of the DNA with 0.1 - 100 units of the restriction enzyme, preferably 1 - 3 units of the restriction enzyme per 1 µg of the DNA in a mixture of 2 - 200 mM, preferably 10 - 40 mM Tris-HCl (pH 6.0 - 9.5, preferably pH 7.0 - 8.0), 0 - 200 mM NaCl and 2 -20 mM, preferably 5 - 10 mM $MgCl_2$ at 20 - 70°C (optimal temperature depends on restriction enzymes used) for 15 minutes to 24 hours. Reaction is usually stopped by heating at 55 - 75°C for 5 - 30 minutes, or alternatively by inactivating the restriction enzyme with a reagent such as phenol or diethylpyrocarbonate.

Purification of the DNA fragments formed by digestion with restriction enzymes is carried out by the above-mentioned LGT method or polyacrylamide gel electrophoresis.

Ligation of the DNA fragments is carried out with 0.3 - 10 units of T4 DNA ligase in a mixture of 2 - 200 mM, preferably 10 - 40 mM Tris-HCl (pH 6.1 - 9.5, preferably 7.0 - 8.0), 2 - 20 mM, preferably 5 - 10 mM $MgCl_2$, 0.1 - 10 mM, preferably 0.5 - 2.0 mM ATP and 1 - 50 mM, preferably 5 -10 mM dithiothreitol at 1 - 37°C, preferably 3 - 20°C for 15 minutes to 72 hours, preferably 2 - 20 hours.

The recombinant plasmid DNA formed by the ligation reaction is introduced into Escherichia coli by the transformation method of Cohen, et al. [S.N. Cohen, et al.: Proc. Natl. Acad. Sci. USA 69, 2110 (1972)], if necessary.

Isolation of the recombinant plasmid DNA from Escherichia coli carrying the plasmid DNA is carried out by the method described in Example 1 or the method of Birnboim, et al. [H.C. Birnboim, et al.: Nucleic Acids Res. 7, 1513 (1979)].

Plasmid DNA is digested with 1 - 10 kinds of restriction enzymes and the cleavage sites are examined by agarose gel electrophoresis or polyacrylamide gel electrophoresis. Further, if necessary, the nucleotide sequence of the DNA is determined by the method of Maxam-Gilbert [Proc. Natl. Acad. Sci. 74, 560 (1977)] or by dideoxy chain termination method using M13 phage [J. Messing, et al.: Gene 19, 269 (1985)].

A recombinant plasmid DNA can be prepared under the foregoing conditions.

The HLTD of the present invention can be prepared in the following manner.

That is, Escherichia coli KM430 (deposited with the Fermentation Research Institute, Agency of Industrial Science and Technology on February 1, 1986 as FERM BP-981) is transformed with a plasmid such as pLdTD11, and an Escherichia coli strain carrying pLdTD11 is selected from the ampicillin resistant (referred to as Ap[r] hereinafter) colonies. The Escherichia coli strain carrying pLdTD11 is cultured in a medium to produce HLTD in the culture broth.

As the medium, either a synthetic medium or a natural medium can be used so long as it is suitable for the growth of Escherichia coli and the production of HLTD.

As a carbon source, glucose, fructose, lactose, glycerol, mannitol, sorbitol, etc. may be used. As a nitrogen source, $NH_4Cl$, $(NH_4)_2SO_4$, Casamino acid, yeast extract, polypeptone, meat extract, Bacto-Tryptone, corn steep liquor, etc. may be used. In addition, nutrients such as $K_2HPO_4$, $KH_2PO_4$, NaCl, $MgSO_4$, vitamine $B_1$ and $MgCl_2$ may be used.

Culturing is carried out at pH 5.5 - 8.5 and at 18 - 40°C with aeration and stirring. After culturing for 5 - 90 hours, HLTD accumulates in cultured cells. The cells are collected from the culture broth, disrupted by ultrasonic treatment and subjected to centrifugation. HLTD can be separated and purified from the supernatant by 40% saturated ammonium sulfate precipitation [Patrick W. Gray, et al.: Nature 312, 721 (1984)]. The protein concentration can be determined by the method of M.M. Bradford [M.M. Bradford: Analytical Biochemistry 72, 248 (1976)]. The HLTD content in a sample can be determined using chromatoscanner after SDS-polyacrylamide gel electrophoresis by the method of Laemmli [U.K. Laemmli: Nature 227, 680 (1970)]. The cytotoxic activity of HLTD is measured by the murine fibroblast L929 assay [Bharat B. Aggarwal, et al.: J. Biol. Chem. 260, 2345 (1985)].

Examples of the present invention are given below.

Example 2

Construction of recombinant plasmid pLA1:

In this Example, 5 μg of pLT1 (4.7 Kb) obtained by the process of Reference Example 1 was dissolved in 50 μℓ (total volume) of a solution containing 10 mM Tris-HCl (pH 7.5), 7 mM MgCl2 and 6 mM 2-mercaptoethanol (referred to as "Y-0 buffer solution" hereinafter). Then, 10 units of restriction enzyme XhoII (product of Boehringer Mannheim GmbH) was added thereto and cleavage reaction was carried out at 37°C for 2 hours. NaCl was added thereto to make a final concentration of 150 mM. Then, 10 units of restriction enzyme NsiI (product of New England Biolabs) was added thereto, and cleavage reaction was carried out at 37°C for 3 hours. About 0.3 μg of a DNA fragment of about 750 bp containing a large portion of the human LT DNA (XhoII-NsiI fragment) was obtained from the reaction solution by LGT method.

Separately, 20 μg of pLT1 was dissolved in 200 μℓ (total volume) of a solution containing 10 mM Tris-HCl (pH 7.5), 7 mM MgCl2, 6 mM 2-mercaptoethanol and 50 mM NaCl (referred to as "Y-50 buffer solution" hereinafter). and 40 units of restriction enzyme HaeIII (product of Takara Shuzo Co.; the restriction enzymes used hereinafter are all products of Takara Shuzo Co., unless otherwise specified) was added thereto. Cleavage reaction was carried out at 37°C for 2 hours. Then, NaCl was added thereto to make a final concentration of 150 mM, and 40 units of NsiI was added thereto. Cleavage reaction was carried out at 37°C for 3 hours. About 40 ng of a DNA fragment of about 50 bp containing the N-terminal region of the mature human LT (HaeIII-NsiI fragment) was obtained from the reaction solution by polyacrylamide gel electrophoresis.

On the other hand, 3 μg of pGEL1 (3.4 Kb) was dissolved in 30 μℓ (total volume) of a solution containing 10 mM Tris-HCl (pH 7.5), 7 mM MgCl2, 6 mM 2-mercaptoethanol and 100 mM NaCl (referred to as "Y-100 buffer solution" hereinafter), and 6 units each of restriction enzymes StuI and BglII were added thereto. Cleavage reaction was carried out at 37°C for 3 hours. About 1.0 μg of a DNA fragment of about 2.3 Kb containing Apr gene (StuI-BglII fragment) was obtained from the reaction solution by LGT method.

Then, 0.2 μg of the XhoII-NsiI fragment (about 750 bp) derived from pLT1, 20 ng of the HaeIII-NsiI fragment (about 50 bp) derived from pLT1 and 0.6 μg of the StuI-BglII fragment (about 2.3 Kb) derived from pGEL1, which were obtained above, were dissolved in 20 μℓ (total volume) of a buffer solution containing 20 mM Tris-HCl (pH 7.6), 10 mM MgCl2, 10 mM dithiothreitol and 1 mM ATP (referred to as "T4 ligase buffer solution" hereinafter), and 2 units of T4 DNA ligase (product of Takara Shuzo Co.; the same shall apply hereinafter) was added to the solution. Reaction was carried out at 4°C for 18 hours.

Escherichia coli KM430 strain was transformed with the thus obtained recombinant plasmid DNA by the method of Cohen, et al. [S.N. Cohen, et al.: Proc. Natl. Acad. Sci., USA 69, 2110 (1972)] (this method is used for transformation of Escherichia coli hereinafter) to obtain an Apr colony. Plasmid DNA was separated and purified from the transformant by the known method [H.C. Birnboim, et al.: Nucleic Acids Res., 7, 1513 (1979)] (this method is used for preparation of plasmid DNA hereinafter), and was cleaved with restriction enzymes such as StuI to make structural analysis. As a result, it was found that the desired plasmid was obtained. The recombinant plasmid is called pLA1.

Example 2

Construction of LT expression plasmid pLSA1:

Escherichia coli KM430 strain carrying pLA1 (3.1 Kb) obtained in Example 1 was cultured, and pLA1 DNA was prepared from the cultured cells according to the method of Birnboim, et al. Then, 3 μg of the obtained PLA1 DNA was dissolved in 30 μℓ of Y-100 buffer solution, and 3 units each of StuI and BglII were added thereto. Cleavage reaction was carried out at 37°C for 3 hours, and about 0.5 μg of a DNA fragment of about 790 bp containing a large portion of human LT gene (StuI-BglII fragment) was obtained from the reaction solution by LGT method.

Separately, 3 μg of pKYP10 prepared according to the method disclosed in Japanese Published Unexamined Patent Application No. 110600/83 was dissolved in 30 μℓ of Y-100 buffer solution, and 6 units each of restriction enzymes BanIII (product of TOYOBO Co.) and PstI were added thereto. Cleavage reaction was carried out at 37°C for 3 hours, and about 0.6 μg of a DNA fragment of about 1.1 Kb containing a tryptophan promoter (Ptrp) (BanIII-PstI fragment) was obtained from the reaction solution by LGT method.

Further, 2 μg of pGEL1 (3.4 Kb) was dissolved in 20 μℓ of Y-100 buffer solution, and 4 units each of restriction enzymes HindIII, BamHI and PstI were added thereto. Cleavage reaction was carried out at 37°C for 3 hours. About 0.7 μg of a DNA fragment of about 1.7 Kb containing a lipoprotein gene-derived terminator (PstI-BamHI fragment) was obtained from the reaction solution by LGT method.

The following DNA linker was synthesized in order to furnish the sequence from Leu (CTA) at the N-terminal of the mature human LT polypeptide to the second base (GG) of Gly (GGC) as the fifth amino acid thereof and also an initiation codon (ATG) necessary for the expression and to adjust the distance between the SD sequence downstream from Ptrp and the ATG to an appropriate length of 6 to 18 bp.

```
BanIII                27-mer          blunt end
                    Met Leu Pro Gly Val
5'-|CGATAAGCTT [ATG][CTA][CCA][GGA][GTA] GG|-3'
 3'-|TATTCGAA TAC GAT GGT CCT CAT CC|-5'
                    25-mer
```

First, two single stranded DNAs of 27-mer and 25-mer were synthesized by a conventional triester method [R. Crea, et al.: Proc. Natl. Acad. Sci., 75, 5765 (1978)]. Then, 20 picomoles each of the 27-mer and 25-mer DNAs were dissolved in 40 μℓ (total volume) of a solution containing 50 mM Tris-HCl (pH 7.5), 10 mM MgCl₂, 5 mM dithiothreitol, 0.1 mM EDTA and 1 mM ATP. 6 units of T4 polynucleotide kinase (product of Takara Shuzo Co.) was added and phosphorylation reaction was carried out at 37°C for 60 minutes.

0.3 μg of the pLA1-derived StuI-BglII fragment (about 790 bp), 0.4 μg of the BanIII-PstI fragment (about 1.1 Kb) of expression vector pKYP10, and 0.6 μg of the pGEL1-derived PstI-BamHI fragment (about 1.7 Kb), which were obtained above, were dissolved in 25 μℓ of T4 ligase buffer solution. About 1 picomole of the DNA linker mentioned above was added to the solution, and 6 units of T4 DNA ligase was further added thereto. Ligation reaction was carried out at 4°C for 18 hours.

Escherichia coli KM430 strain was transformed with the reaction mixture containing the recombinant plasmid to obtain an Apr colony. Plasmid DNA was recovered from the cultured cells of the colony. The structure of the thus obtained plasmid was determined by agarose gel electrophoresis after cleavage with restriction enzymes EcoRI, BanIII, PstI, HindIII, and BglII. The plasmid is called pLSA1. The nucleotide sequence around BanIII and HindIII sites in pLSA1 was found as follows by the method of Maxam-Gilbert [A.M. Maxam, et al.: Proc. Natl. Acad. Sci., USA 74, 560 (1977)].

```
BanIII  HindIII
                   Met Leu Pro Gly Val Gly Leu
|CG ATA|AGCT| T ATG CTA CCA GGA GTA GGC CTC
```

### Example 3

Construction of recombinant plasmid pLC2:

First, 3 μg of pLT1 (4.7 Kb) obtained according to the method in Reference Example 1 was dissolved in 30 μℓ of Y-50 buffer solution, and 6 units each of restriction enzymes XmnI (product of New England Biolabs) and HindIII were added thereto. Cleavage reaction was carried out at 37°C for 3 hours. About 0.6 μg of a DNA fragment of about 1.5 Kb containing human LT DNA (XmnI-HindIII fragment) was obtained from the reaction solution by LFT method.

Separately, 1 μg of pUC9 (2.7 Kb) was dissolved in 20 μℓ of Y-100 buffer solution, and 2 units each of restriction enzymes HincII and HindIII were added thereto. The mixture was subjected to cleavage reaction at 37°C for 3 hours. About 0.5 μg of a DNA fragment of about 2.7 Kb (HincII-HindIII fragment) was obtained from the reaction solution of LGT method.

Then, 0.3 μg of the pLT1-derived XmnI-HindIII fragment (about 1.5 Kb) and 0.3 μg of the pUC9-derived HincII-HindIII fragment (about 2.7 Kb) thus obtained were dissolved in 20 μℓ (total volume) of T4 ligase buffer solution, and one unit of T4 DNA ligase was added thereto. Ligation reaction was carried out at 4°C for 18 hours.

Escherichia coli KM430 strain was transformed with the thus obtained mixture of the recombinant plasmids, and Aprcolonies were obtained. Plasmid DNA pLC2 was recovered from the cultured cells of the colonies. The structure of pLC2 was confirmed by agarose gel electrophoresis after cleavage with restriction enzymes BamHI, HindIII and PstI.

### Example 4

Construction of plasmid pLdTD11 and pLdTD12 coding for the polypeptides wherein the N-terminal region of the mature human LT is deleted:

Escherichia coli KM430 strain carrying pLC2 (4.2 Kb) obtained in Example 3 was cultured, and pLC2 DNA was prepared from the cultured cells according to the method of Birnboim, et al. Then, 20 μg of the obtained pLC2 DNA was dissolved in 200 μℓ of Y-100 buffer solution, and 40 units of restriction enzyme ApaI (product of Boehringer Mannheim GmbH) was added. Cleavege reaction was carried out at 37°C for 3 hours. To 50 μℓ of

the reaction solution containing 5 µg of the DNA, were added 20 µℓ of 5-fold concentrated BAL31 buffer solution [100 mM Tris-HCl (pH 8.1), 3M NaCl, 60 mM CaCl₂, 60 mM MgCl₂, and 5 mM EDTA], 30 µℓ of water and 0.4 unit of nuclease BAL31 [product of Bethesda Research Laboratories (BRL)], and reaction was carried out at 30°C for 20 seconds. BAL31 has the activity of exonuclease with digests from the end of a DNA molecule and about 60 bp of DNA from the ApaI site were digested under the conditions described above. The reaction solution was subjected to phenol extraction and chloroform extraction, and DNA was recovered therefrom by ethanol precipitation. Then, 3.0 µg of the recovered pLC2-ApaI-BAL31 cleaved fragment was dissolved in 30 µℓ of Y-100 buffer solution, and 4 units of BamHI was added thereto. Cleavage reaction was carried out at 37°C for 2 hours. and about 0.3 µg of a DNA fragment of about 650 bp (pLC2-ApaI-BAL31-BamHI fragment) was recovered from the reaction solution by LGT method.

Separately, 5.0 µg of ATG vector pTrS20 (3.8 Kb) obtained according to the method of Reference Example 2 was dissolved in 50 µℓ of Y-0 buffer solution, and 16 units of SacI was added thereto. Cleavage reaction was carried out at 37°C for 3 hours. After phenol extraction and chloroform extraction, about 4.0 µg of a DNA fragment was purified and recovered by ethanol precipitation. Then, 4.0 µg of the DNA fragment of 3.8 Kb was dissolved in a solution containing 50 mM Tris-HCl (pH 7.8), 7 mM MgCl₂ and 6 mM 2-mercaptoethanol, and dATP, dTTP, dCTP and dGTP were added thereto to make 1 mM each (total volume 80 µℓ). Furthermore, 5 units of Klenow fragment (product of Takara Shuzo Co.) was added thereto, and reaction was carried out at room temperature for one hour to digest the protruding end. After phenol extraction and chloroform extraction, 3.0 µg of a DNA fragment was recovered by ethanol precipitation. Then, 3.0 µg of the DNA fragment was dissolved in 30 µℓ (total volume) of Y-100 buffer solution, and 6 units of PstI was added thereto. Cleavage reaction was carried out at 37°C for 3 hours, and about 0.5 µg of a DNA fragment of about 1.1 Kb containing Ptrp (pTrS20-SacI-Klenow fragment-PstI fragment) was removed from the reaction solution by LGT method.

Further, 2 µg of pGEL1 was dissolved in 20 µℓ of Y-100 buffer solution, and 4 units each of HindIII, BamHI and PstI were added thereto. Cleavage reaction was carried out at 37°C for 3 hours, and about 0.7 µg of a DNA fragment of about 1.7 Kb containing the lipoprotein gene-derived terminator (PstI-BamHI fragment) was obtained from the reaction solution by LGT method.

Then, 0.2 µg of the pLC2-ApaI-BAL31-BamHI fragment (about 650 bp), 0.4 µg of the pTrS20-SacI-Klenow fragment-PstI fragment (about 1.1 Kb) and 0.6 µg of the pGEL1-derived PstI-BamHI fragment (about 1.7 Kb) thus obtained were dissolved in 20 µℓ (total volume) of T4 ligase buffer solution, and one unit of T4 DNA ligase was added thereto. Ligation reaction was carried out at 4°C for 18 hours.

Escherichia coli KM430 strain was transformed with the thus obtained mixture of recombinant plasmids, and plasmid DNAs were recovered from the cultured cells of the resulting Apʳ colonies. These plasmids are called pLdTD11 and pLdTD12, as shown in Fig. 4.

The structures of these plasmids were confirmed by agarose gel electrophoresis after cleavage with restriction enzymes BanIII, HindIII, EcoRI and PstI. It was confirmed by dideoxy chain termination method using M13 phage that the sequences around the N-terminal of human LT structural gene in the plasmids were as follows:

```
                Met Ala His Ser Thr Leu
pLdTD11  -  ATG GCC CAC AGC ACC CTC

                Met Gln Thr Ala Arg Gln
pLdTD12  -  ATG CAG ACT GCC CGT CAG
```

It was also confirmed that HLTD encoded by pLdTD11 [the derivative is referred to as LT (Δ1-22)] had the structure wherein 22 amino acids from Leu at the N-terminal to Leu at the 22nd position of the mature human LT polypeptide were deleted, and started from Ala at the 23rd position. Likewise, it was confirmed that HLTD encoded by pLdTD12 [the derivative is referred to as LT (Δ1-11)] had the structure wherein 11 amino acids from the N-terminal to Ala at the 11th position were deleted, and started from glutamine (Gln) at the 12th position.

### Example 5

Construction of plasmid pLdTA2 coding for the polypeptide wherein the N-terminal region of the mature human LT is deleted:

First, 3 µg of pLC2 DNA obtained in Example 3 was dissolved in 30 µℓ (total volume) of a solution containing 10 mM Tris-HCl (pH 7.5), 7 mM MgCl₂, 6 mM 2-mercaptoethanol and 150 mM NaCl (hereinafter referred to as "Y-150 buffer solution"), and 6 units each of NsiI and BamHI were added thereto. Cleavage reaction was carried out at 37°C for 3 hours, and about 0.3 µg of a DNA fragment of about 60 bp containing a large portion of the human LT gene (NsiI-BamHI fragment) was obtained from the reaction solution by LGT method.

Separately, 3 µg of ATG expression vector pTrS10 (4.2 Kb) obtained according to the method of Reference Example 3 was dissolved in 30 µℓ of Y-150 buffer solution, and 6 units each of NsiI and PstI were added thereto. Cleavage reaction was carried out at 37°C for 3 hours, and about 0.6 µg of a DNA fragment of about 1.1 Kb containing Ptrp (NsiI-PstI fragment) was obtained from the reaction solution by LGT method.

Further, 2 μg of pGEL1 was dissolved in 20 μℓ of Y-100 buffer solution, and 4 units each of HindIII, BamHI and PstI were added thereto. Cleavage reaction was carried out at 37°C for 3 hours, and about 0.7 μg of a DNA fragment of about 1.7 Kb containing a lipoprotein gene-derived terminator (PstI-BamHI fragment) was obtained from the reaction solution by LGT method.

Then, 0.2 μg of the pLC2-derived NsiI-BamHI fragment (about 620 bp), 0.4 μg of the pTrS10-derived NsiI-PstI fragment (about 1.1 Kb) and 0.6 μg of the pGEL1-derived PstI- BamHI fragment (about 1.7 Kb) thus obtained were dissolved in 20 μℓ (total volume) of T4 ligase buffer solution, and one unit of T4 DNA ligase was added thereto. Ligation reaction was carried out at 4°C for 18 hours.

Escherichia coli KM430 strain was transformed with the thus obtained mixture of recombinant plasmids to obtain an Ap$^r$ colony. Plasmid DNA was recovered from the cultured cells of the colony, and pLdTA2 was obtained. The structure of pLdTA2 was confirmed by agarose gel electrophoresis after cleavage with restriction enzymes NsiI, EcoRI, PstI, BanIII and BamHI.

## Example 6

Production of HLTD by Escherichia coli strains carrying pLSA1, pLdTD11, pLdTD12 and pLdTA2:

Escherichiacoli KM430 strains respectively carrying recombinant plasmids pLSA1, PLdTD11, pLdTD12 and pLdTA2 obtained in Examples 2, 4 and 5, which are named ELSA1, ELdTD11, ELdTD12 and ELdTA2 respectively, were cultured at 37°C for 18 hours in LG medium prepared by dissolving 10g of Bacto-Tryptone, 5g of yeast extract, 5g of NaCl and 1g of glucose in 1ℓ of water and adjusting the pH to 7.0 with NaOH. 0.5 mℓ of the culture medium was inoculated into 10 mℓ of MCG medium (0.6% $Na_2HPO_4$, 0.3% $KH_2PO_4$, 0.5% NaCl, 0.5% Casamino acid, 1 mM $MgSO_4$, and 4 μg/mℓ vitamin $B_1$, pH 7.2) containing 25 μg/mℓ tryptophan and 50 μg/mℓ ampicillin and culturing was carried out at 30°C for 4 to 8 hours. Then, 3β-indoleacrylic acid (hereinafter referred to as "IAA") which is an inducer of tryptophan operon was added thereto to make a final concentration of 10 μg/mℓ, and culturing was continued for 2 to 12 hours. The culture medium was centrifuged at 8,000 rpm for 10 minutes to collect the cells, and the cells were washed with a buffer solution containing 30 mM NaCl and 30 mM Tris-HCl (pH 7.5). The washed cells were suspended in 3 mℓ of the said buffer solution, and subjected to ultrasonic disruption at 0°C (Branson Sonic Power Company, Sonifier cell disruptor 200, output control 2, 10 minutes). The disrupted cell suspension was centrifuged at 15,000 rpm for 30 minutes to obtain a supernatant. HLTD was separated and purified from the supernatant by 40% saturated ammonium sulfate precipitation. Protein concentration was determined according to the method of M.M. Bradford. HLTD content in the sample was determined using chromatoscanner (two-wavelength TLC scanner: Model CS-930, product of Shimazu Seisakusho) after SDS polyacrylamide gel electrophoresis according to the method of Laemmli. The cytotoxic activity of HLTD was measured according to the murine fibroblast L929 assay.

The results are shown in Table 1.

## Table 1

| Strain | Plasmid contained | Product encoded by plasmid | Relative activity* |
|--------|-------------------|----------------------------|--------------------|
| ELSA1 | pLSA1 | LT (Intact) | 1.0 |
| ELdTD11 | pLdTD11 | LT (Δ1-22) | 2.5 |
| ELdTD12 | pLdTD12 | LT (Δ1-11) | 1.5 |
| ELdTA2 | pLdTA2 | LT (Δ1-19) | 1.3 |

\* Expressed as ratio of activity relative to mature (intact) LT polypeptide.

ELdTD11. ELdTD12 and ELdTA2 were deposited with the Fermentation Research Institute, Agency of Industrial Science and Technology on January 10, 1986 under numbers FERM BP-963, 964 and 962, respectively.

Reference Example 1

Isolation of Plasmid pLT1 carrying human LT cDNA:

(1) Preparation of poly (A) RNA from LukII cells:

RNA containing poly (A) was prepared from LukII human lymphoblastoid cell line according to the guanidine thiocyanate-lithium chloride method [Cathala, et al.: DNA $\underline{2}$, 329 (1983)] in the following manner.

LukII human lymphoblastoid cell line [Berish Y. Rubin, et al.: Proc. Natl. Acad. Sci. USA $\underline{82}$, 6637 (1985)] was inoculated into 1$\ell$ of RPMI1640 medium (product of Nissui Seiyaku Co.) containing 5% fetal calf serum and 1 mM N-2-hydroxyethyl-piperazine-N'-2-ethanesulfonic acid (HEPES) to make a concentration of 8 x $10^5$/m$\ell$, and propagated. A spinner culture bottle was used for the culturing. After culturing at 37°C for 48 hours, the cells were collected by centrifugation, transferred into 1$\ell$ of a fresh RPMI1640 medium containing 5% fetal calf serum, 10 ng/ml phorbol myristate acetate (PMA) and 1 mM HEPES, and further cultured at 37°C for 48 hours. Then, the cells were collected from a portion (250 m$\ell$) of the cell suspension by centrifugation at 1,100 x G and at 4°C for 10 minutes, washed with 80 m$\ell$ of a phosphate buffer, and solubilized in 10 m$\ell$ of a solution comprising 5M guanidine thiocyanate, 10 mM EDTA, 50 mM Tris-HCl (pH 7) and 8% (V/V) 2-mercaptoethanol in a voltex mixer. The solubilized product was transferred into a centrifuge tube, and 80 m$\ell$ of a 4M LiC1 solution was added thereto. The mixture was stirred, and then allowed to stand at 4°C for 20 hours. After centrifugation at 9,000 rpm for 90 minutes using Hitachi RPR10 rotor, RNA was recovered as precipitates. The RNA precipitates were suspended in 50 m$\ell$ of a solution comprising 4M urea and 2M lithium chloride, and the suspension was centrifuged at 9,000 rpm for 60 minutes using Hitachi RPR10 rotor. Then, RNA was again recovered as precipitates, and the precipitates were dissolved in 10 m$\ell$ of a solution comprising 0.1% sodium laurylsulfate, 1 mM EDTA and 10 mM Tris-HCl (pH 7.5). After extraction with phenol-chloroform, RNA was recovered by ethanol precipitation. About 2.5 mg of the thus obtained RNA was dissolved in 1 m$\ell$ of a solution containing 10 mM Tris-HCl (pH 8.0) and 1 mM EDTA, and incubated at 65°C for 5 minutes. Then, 0.1 m$\ell$ of 5M NaCl was added thereto. The mixture was subjected to column chromatography on oligo (dT) cellulose (product of P-L Biochemical; column volume: 0.5 m$\ell$). The absorbed mRNA having poly (A) was eluted with a solution comprising 10 mM Tris-HCl (pH 7.5) and 1 mM EDTA, whereby about 100 μg of mRNA having poly (A) was obtained.

(2) cDNA synthesis and insertion of the DNA into a vector;

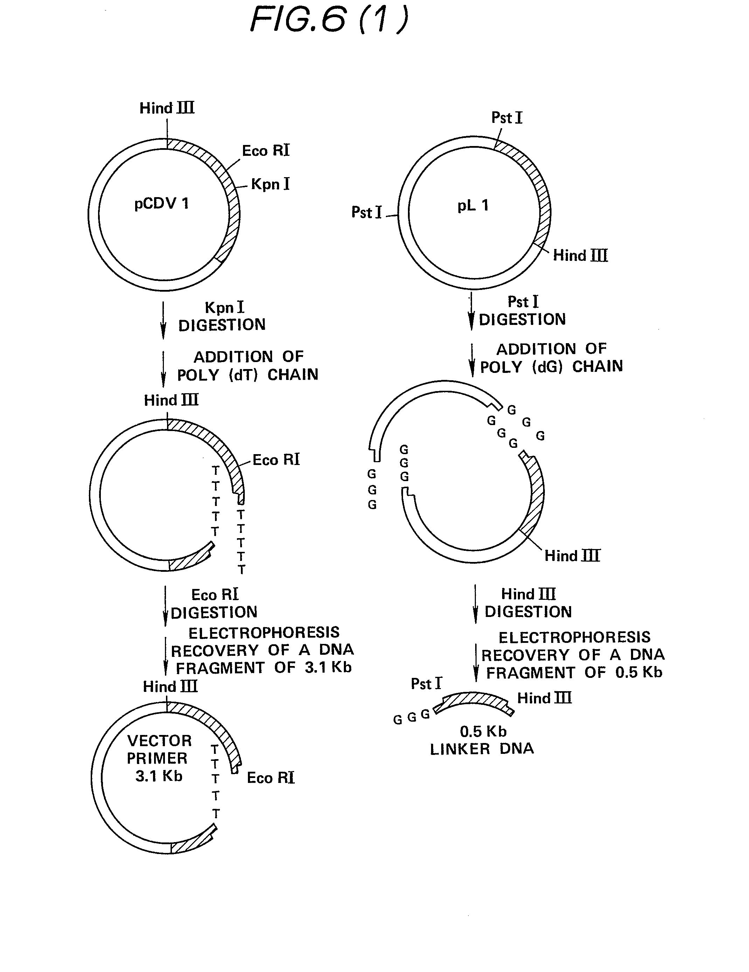

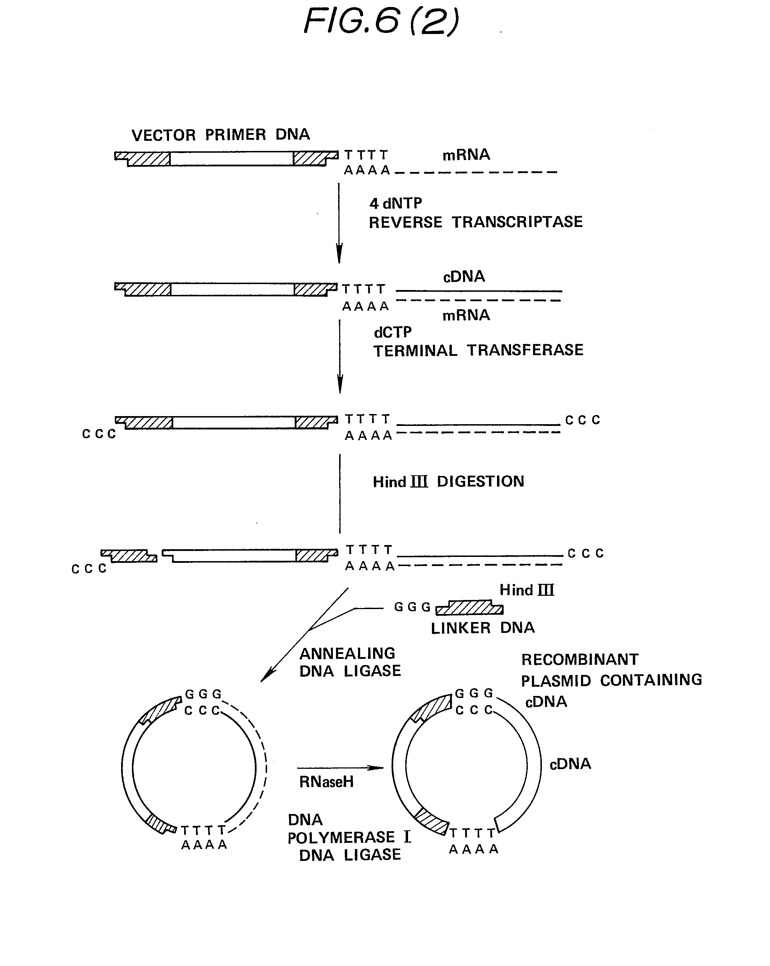

Synthesis of a cDNA and construction of a recombinant plasmid wherein the cDNA is incorporated were carried out according to the method of Okayama-Berg [Mol. Cell. Biol. $\underline{2}$, 161 (1982)]. The outline of the steps is shown in Fig. 6.

First, 400 μg of pcDV1 [Okayama & Berg: Mol. Cell. Biol. $\underline{3}$, 280 (1983)] was added to 300 μ$\ell$ of a solution comprising 10 mM Tris-HCl (pH 7.5), 6 mM MgCl$_2$ and 10 mM NaCl, and 500 units of KpnI was added thereto. Reaction was carried out at 37°C for 6 hours to make cleavage at the KpnI site in the plasmid. After phenol-chloroform extraction, DNA was recovered by ethanol precipitation. About 200 μg of the DNA cleaved with KpnI was added to 200 μ$\ell$ of a solution prepared by adding dTTP to a buffer solution comprising 40 mM sodium cacodylate, 30 mM Tris-HCl (pH 6.8), 1 mM CaCl$_2$ and 0.1 mM dithiothreitol (hereinafter referred to as DTT) (the buffer solution is hereinafter referred to as "TdF buffer solution") to make a dTTP concentration of 0.25 mM, and further, 81 units of terminal deoxynucleotidyl transferase (hereinafter referred to as TdT, product of P-L Biochemicals) was added thereto. The mixture was subjected to reaction at 37°C for 11 minutes, whereby a poly (dT) chain (about 67-mer) was added to the 3'terminal at the KpnI-cleaved site of pcDV1. About 100 μg of pcDV1 DNA having poly (dT) chain was recovered from the solution by phenol-chloroform extraction and ethanol precipitation. Then, the DNA was added to 150 μ$\ell$ of a buffer solution comprising 10 mM Tris-HCl (pH 7.5), 6 mM MgCl$_2$ and 100 mM NaCl, and 360 units of EcoRI was added thereto. The mixture was subjected to reaction at 37°C for 2 hours, and the reaction product was treated by LGT method, whereby a DNA fragment of about 3.1 Kb was recovered. About 60 μg of poly (dT) chain-added pcDV1 was obtained. The DNA was dissolved in 500 μ$\ell$ of a solution comprising 10 mM Tris-HCl (pH 8.0) and 1 mM EDTA, incubated at 65°C for 5 minutes, and ice-cooled. Then, 50 μ$\ell$ of 5M NaCl was added thereto, and the mixture was subjected to column chromatography on oligo (dA) cellulose (product of Collaborative Research). DNAs having a poly (dT) chain with a sufficient length were adsorbed on the column and eluted with a solution comprising 10 mM Tris-HCl (pH 8.0) and 1 mM EDTA, whereby 27 μg of poly (dT) chain-added pcDV1 (hereinafter referred to as "vector primer") was obtained.

Then, a linker DNA was prepared.

About 14 μg of pL1 [Okayama & Berg: Mol. Cell. Biol. $\underline{3}$, 280 (1983)] was added to 200 μ$\ell$ of a buffer solution comprising 10 mM Tris-HCL (pH 7.5), 6 mM MgCl$_2$ and 50 mM NaCl, and 50 units of PstI was added thereto. The mixture was subjected to reaction at 37°C for 4 hours to cleave pL1 DNA at the PstI site. The reaction product was subjected to phenolchloroform extraction and then to ethanol precipitation, whereby about 13 μg of pL1 DNA cleaved with PstI was recovered. About 13 μg of the DNA was added to 50 μ$\ell$ of a solution prepared by adding dGTP to TdT buffer solution to a final concentration of 0.25 mM, and further, 54 units of TdT (product of P-L Biochemicals) was added thereto. The mixture was subjected to incubation at 37°C for 13 minutes to add a (dG) chain (about 14-mer) to the 3'terminal at the PstI-cleaved site of pL1. After phenol-chloroform extraction, DNA was recovered by ethanol precipitation. Then, the DNA was added to 100

μℓ of a buffer solution comprising 10 mM Tris-HCl (pH 7.5), 6 mM MgCl₂ and 60 mM NaCl, and 80 units of HindIII was added thereto. The mixture was subjected to incubation at 37°C for 3 hours, whereby pL1 DNA was cleaved at the HindIII site. The reaction product was fractionated by agarose gel electrophoresis and a DNA fragment of about 0.5 Kb was recovered by DEAE paper method [Dretzen, et al.: Anal. Biochem., 112, 295 (1981)], whereby a linker DNA with the oligo (dG) chain (hereinafter referred to merely as "linker DNA") was obtained.

About 2 μg of the poly (A) RNA and about 1.4 μg of the vector primer thus obtained were dissolved in 22.3 μℓ of a solution comprising 50 mM Tris-HCl (pH 8.3), 8 mM MgCl₂, 30 mM KCl, 0.3 mM DTT, 2 mM dNTP (dATP, dTTP, dGTP and dCTP) and 10 units of ribonuclease inhibitor (product of P-L Biochemicals), and 10 units of reverse transcriptase (product of Seikagaku Kogyo Co.) was added thereto. The mixture was subjected to incubation at 41°C for 90 minutes to synthesize a DNA complementary to the mRNA. The reaction product was subjected to phenol-chloroform extraction and then to ethanol precipitation, whereby a vector primer DNA with RNA-DNA double-stranded chain was recovered. The DNA was dissolved in 20 μℓ of TdT buffer solution containing 66 μM dCTP and 0.2 μg of poly (A), and 14 units of TdT (product of P-L Biochemicals) was added thereto. The mixture was subjected to incubation at 37°C for 2 minutes to add a (dC) chain (20-mer) to the 3'terminal of the cDNA. The reaction product was subjected to phenol-chloroform extraction, and then to ethanol precipitation, whereby a (dC) chain-added cDNA-vector primer DNA was recovered. The DNA was dissolved in 400 μℓ of a solution comprising 10 mM Tris-HCl (pH 7.5), 6 mM MgCl₂ and 60 mM NaCl, and 20 units of HindIII was added thereto. The mixture was subjected to incubation at 37°C for 2 hours to cleave the DNA at the HindIII site. The reaction product was subjected to phenol-chloroform extraction, and to ethanol precipitation, whereby 0.5 picomole of a (dC) chain-added cDNA-vector primer DNA was obtained. Then, 0.2 picomole of the DNA and 0.4 picomole of the linker DNA were dissolved in 100 μℓ of a solution comprising 10 mM Tris-HCl (pH 7.5), 0.1M NaCl and 1 mM EDTA, and the mixture was subjected to incubation at 65°C for 10 minutes, at 42°C for 25 minutes and at 0°C for 30 minutes. To the reaction solution were added Tris-HCl (pH 7.5), MgCl₂, (NH₄)₂SO₄, KCl and β-NAD to make 1000 μℓ of a solution containing 20 mM Tris-HCl (pH 7.5), 4 mM MgCl₂, 10 mM (NH₄)₂SO₄, 0.1M KCl and 0.1 mM β-NAD. Then, 25 units of Escherichia coli DNA ligase (product of New England Biolabs) was added to the reaction solution, and the mixture was subjected to incubation at 11°C for 18 hours. dNTP and β-NAD were added to the reaction solution to concentrations of 40 μM and 0.15 mM, respectively, and 10 units of Escherichia coli DNA ligase, 20 units of Escherichia coli DNA polymerase I (product of P-L Biochemicals) and 10 units of Escherichia coli ribonuclease H (product of P-L Biochemicals) were added thereto. The mixture was subjected to incubation at 12°C for one hour and then at 25°C for one hour. By the reaction, cyclization of the recombinant DNA containing cDNA and replacement of the RNA part of RNA-DNA double-stranded chain with DNA were carried out, and a recombinant plasmid of complete double-stranded DNA was formed.

(3) Selection of recombinant DNA containing human LT cDNA:

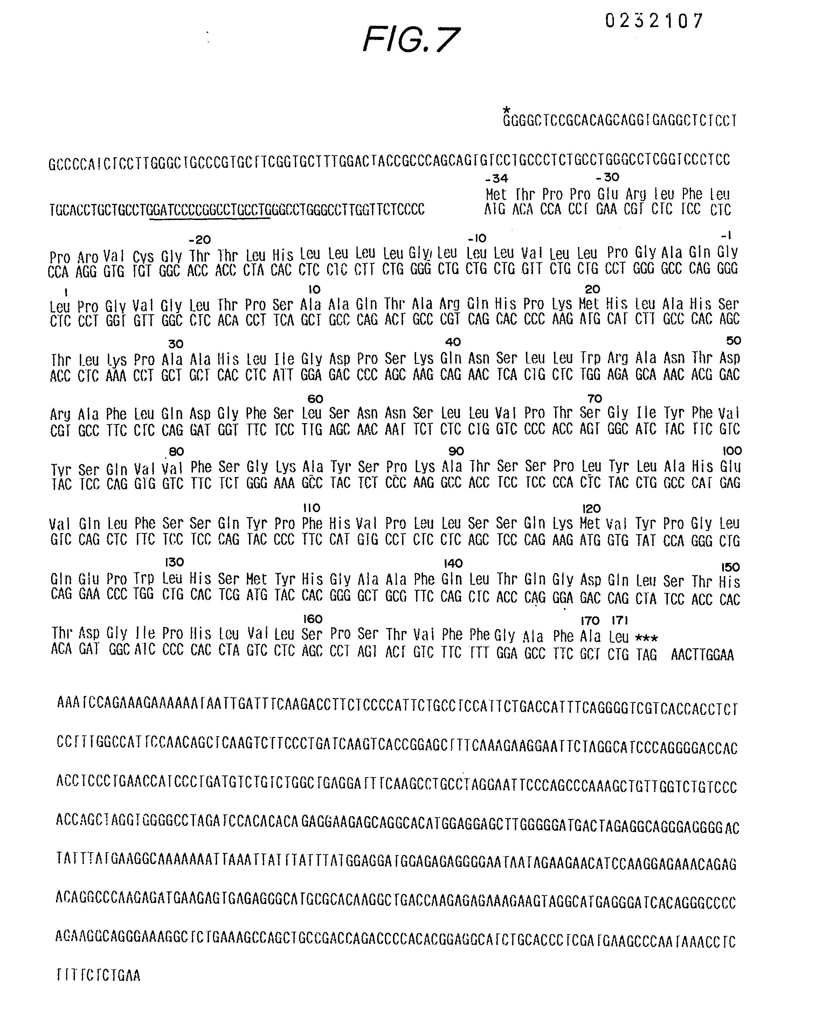

Escherichia coli C600SF8 strain [Cameron: Proc. Natl. Acad. Sci., USA 72, 3416 (1975)] was transformed with the recombinant plasmid obtained in (2) according to the method of Scott, et al. [Katsuya Shigesada: Saibo Kogaku 2, 616 (1983)]. About 30,000 colonies thus obtained were immobilized on nitrocellulose filters. A synthetic DNA of 17 bases, 5'-GATCCCCGGCCTGCCTG-3', which coincides with the nucleotide sequence of a part of 5'-untranslated region of the human LT cDNA isolated by Genentech, Inc. [Patrick W. Gray, et al.: Nature 312, 721 (1984)] and corresponds to the underlined part of the nucleotide sequence of a human LT cDNA of plasmid pLT1 isolated by the present inventor shown in Fig. 7 was labeled with 32p and used as a probe. One strain which strongly hybridized with this probe at 52°C was selected according to the Grunstein-Hogness method [Proc. Natl. Acad. Sci. USA 72, 3961 (1975)]. The complete nucleotide sequence of the cDNA of plasmid pLT1 carried by the strain was determined according to the dideoxy chain termination method using M13 phage (Fig. 7). It was found that the cDNA of pLT1 coded for human LT.

Reference Example 2

Construction of ATG Vector pTrS20:

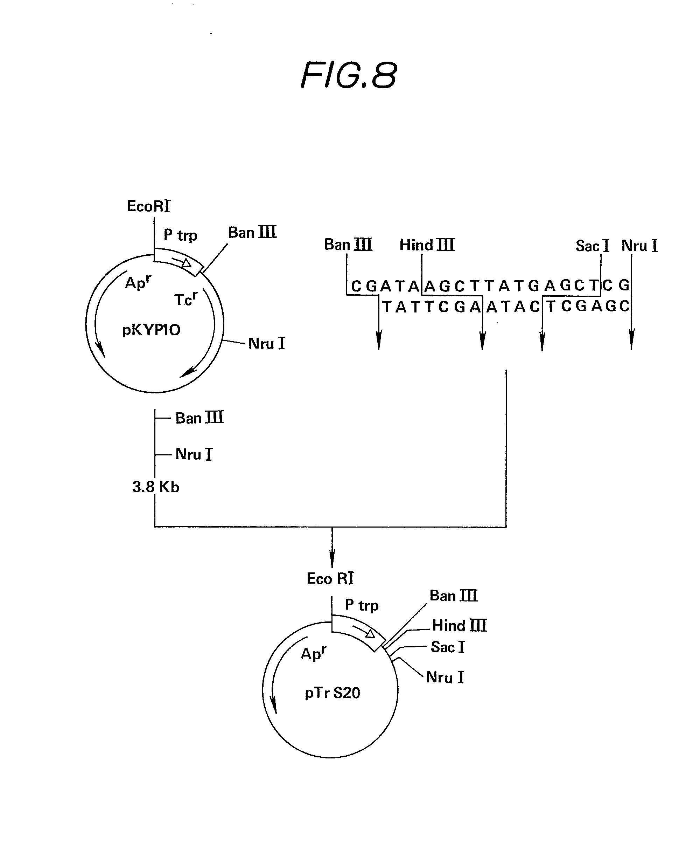

ATG vector pTrS20 having a distance of 14 bases between the SD sequence and the ATB initiation codon and having a SacI site just after the ATG codon was constructed according to the procedure shown in Fig. 8.

First, 3 μg of pKYP10 prepared according to the procedure disclosed in Japanese Published Unexamined Patent Application No. 110600/83 was dissolved in 30 μℓ of Y-100 buffer solution, and 6 units each of restriction enzymes BanIII and NruI (product of new England Biolabs) were added thereto. The mixture was subjected to cleavage reaction at 37°C for 3 hours, and about 0.5 μg of a DNA fragment of about 3.8 Kb containing Ptrp (BanIII-NruI fragment) was obtained from the reaction solution by LGT method.

Separately, the following DNA linker was synthesized according to the triester method to provide an ATG initiation codon downstream from Ptrp.

```
  BanIII        HindIII          Met           SacI  NruI
5'-|C G A T A|A G C T T A T G A G C T|C G|- 3'  (19-mer)
   3'-|T A T T C G A|A T A C T C G A|G C|- 5'  (17-mer)
```

10 picomoles each of synthesized 19-mer and 17-mer DNAs were dissolved in 20 μℓ (total volume) of a solution containing 50 mM Tris-HCl (pH 7.5). 10 mM MgCl2, 5 mM dithiothreitol, 0.1 mM EDTA and 1 mM ATP, and 3 units of T4 polynucleotide kinase (product of Takara Shuzo Co.) was added thereto. Phosphorylation reaction was carried out at 37°C for 60 minutes.

Then, 0.1 μg of the pKYP10-derived BanIII-NruI fragment (about 3.8 Kb) and about 0.5 picomole of the DNA linker thus obtained were dissolved in 20 μℓ of T4 ligase buffer solution, and 2 units of T4 DNA ligase was added thereto. Ligation reaction was carried out at 4°C for 18 hours.

Escherichia coli HB101 strain [Bolivar, et al.: Gene 2, 75 (1977)] was transformed with the resulting mixture or recombinant plasmids to obtain an Apr colony. A plasmid DNA was recovered from the cultured cells of the colony. The structure of the thus obtained plasmid was confirmed by agarose gel electrophoresis after cleavage with restriction enzymes EcoRI, BanIII, HindIII, SacI and NruI. The plasmid was named pTrS20 (Fig. 8). It was confirmed by the dideoxy chain termination method using M13 phage that the nucleotide sequence around the BanIII and HindIII sites in pTrS20 was as follows.

```
                   BanIII  HindIII         SacI  NruI
SD sequence                        Met
       AAGG  GTAT |CGATA |AGCTT  ATG  AGCT| CG |CGA
```

Reference Example 3

Construction of ATG vector pTrS10:

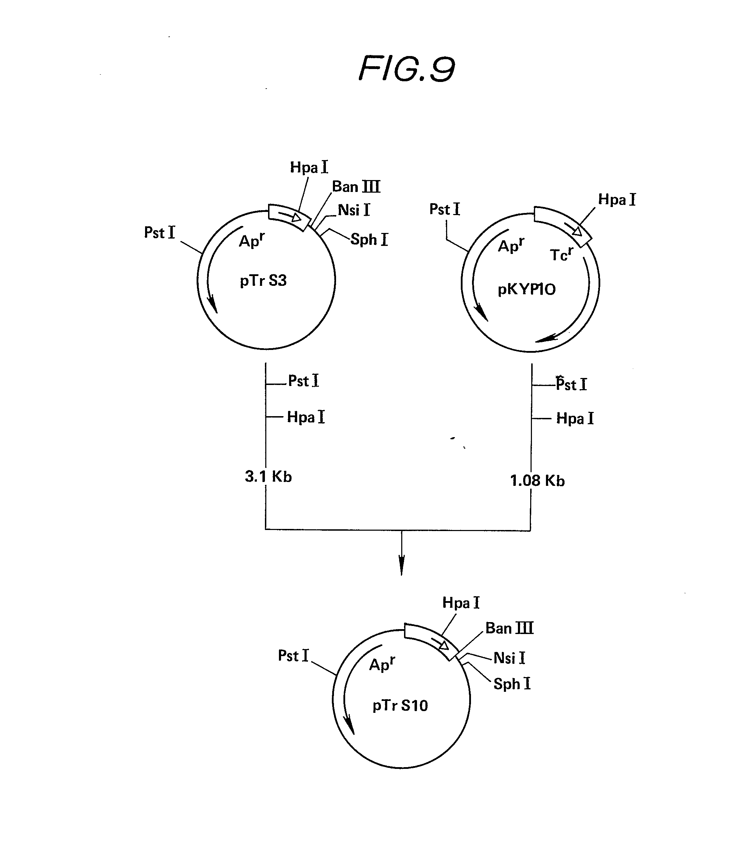

ATG vector pTrS10 having a portable Ptrp fragment of 370 base pairs, a distance of 13 bases between the SD sequence and the ATG initiation codon and an SphI site just after the ATG codon was constructed according to the procedure shown in Fig. 9.

First, 3 μg of pTrS3 prepared according to the method disclosed in Japanese Published Unexamined Patent Application No. 110600/83 was dissolved in 30 μℓ of Y-100 buffer solution, and 6 units each of restriction enzymes PstI and HpaI were added thereto. The mixture was subjected to cleavage reaction at 37°C for 3 hours, and about 2 μg of a DNA fragment of about 3.1 Kb containing a part of Ptrp and the ATG initiation codon (PstI-HpaI fragment) was obtained from the reaction solution by LGT method.

Separately, 3 μg of pKYP10 prepared according to the method disclosed in Japanese Published Unexamined Patent Application No. 110600/83 was dissolved in 30 μℓ of Y-100 buffer solution, and 6 units each of restriction enzymes PstI and HpaI were added thereto. The mixture was subjected to cleavage reaction at 37°C for 3 hours, and about 0.5 μg of a DNA fragment of about 1.08 Kb containing a part of Ptrp (PstI-HpaI fragment) was obtained from the reaction solution by LGT method.

Then, 0.1 μg of the pTrS3-derived PstI-HpaI fragment and 0.1 μg of the pKYP10-derived PstI-HpaI fragment thus obtained were dissolved in 20 μℓ of T4 ligase buffer solution, and 2 units of T4 DNA ligase was added thereto. The mixture was subjected to ligation reaction at 4°C for 18 hours.

Escherichia coli HB101 strain was transformed with the thus obtained mixture of recombinant plasmids to obtain an Apr colony. Plasmid DNA was prepared from the cultured cells of the colony, and subjected to structural analysis using restriction enzymes PstI, HpaI, BanIII, NsiI and SphI. It was confirmed that the desired plasmid pTrS10 was obtained.

**Claims**

1. A human lymphotoxin polypeptide derivative of high LT activity, characterised in that the polypeptide has the amino acid sequence from Leu (No. 1) to Leu (No. 171) as shown in Formula 1, but modified in that certain of the amino acids are missing or are removed.

2. A polypeptide derivative according to claim 1, characterised in that said certain amino acid(s) is or are missing from the mature human lymphotoxin polypeptide at the N-terminal.

3. A polypeptide derivative according to claim 2, characterised in that the missing amino acids are from the first twenty-two amino acids in the amino acid sequence shown in Formula 1.

4. A polypeptide derivative according to claim 3, characterised in that all the first twenty-two amino acids of the sequence are missing.

5. A polypeptide derivative according to claim 3, characterised in that the first eleven amino acids of the sequence are missing.

6. A polypeptide derivative according to claim 3, characterised in that the first nineteen amino acids of the sequence are missing.

7. A DNA coding for a human lymphotoxin polypeptide derivative of high LT activity, characterised in that the DNA codes for the amino acid sequence set forth in Formula 1, running from amino acids Leu (No. 1) to Leu (No. 171), but with at least one of the first twenty-two amino acids missing.

8. A DNA according to claim 7, characterised in that it codes for an amino acid sequence as specified in which either amino acids 1 to 22, 1 to 11 or 1 to 19 are missing.

9. A recombinant plasmid incorporating a DNA fragment coding for a human lymphotoxin polypeptide derivative, characterised in that the DNA codes for a mature human lymphotoxin polypeptide having the amino acid sequence shown in Formula 1, but in which certain selected ones of the amino acid sequence are missing.

10. A recombinant plasmid according to claim 9, characterised in that the DNA fragment codes for an amino acid sequence as specified, and in which at least one of the first twenty-two amino acids are missing.

11. A recombinant plasmid according to claim 10, characterised in that the DNA fragment codes for an amino acid sequence as specified, and in which either amino acids 1 to 22, 1 to 11 or 1 to 19 are missing.

12. A recombinant plasmid according to claim 9, 10 or 11, characterised in that the plasmid DNA has a tryptophan promoter and the said DNA fragment is incorporated into the plasmid DNA downstream from the tryptophan promoter.

13. The recombinant plasmids herein identified as pLdTA2, pLdTD11 and pLdD12.

14. A process for producing a human lymphotoxin polypeptide derivative (HLPD), which comprises culturing a transformed microorganism containing a recombinant plasmid containing a DNA fragment coding for a human lymphotoxin polypeptide derivative and capable of expressing HLPD in the culture medium, producing and accumulating the HLPD in the culture medium, and recovering the product HLPD.

15. A process according to claim 14, characterised in that the microorganism is a transformed Escherichia coli.

16. A process according to claim 14 or 15, characterised in that the microorganism is transformed with a plasmid according to any one of claims 9-13.

17. A biologically pure culture of a transformed microorganism, characterised in that the microorganism is transformed with a recombinant plasmid containing a DNA fragment coding for a human lymphotoxin polypeptide derivative.

18. A biologically pure culture according to claim 17, characterised in that the microorganism is a transformed Escherichia coli.

19. A biologically pure culture of a transformed microorganism according to claim 17 or 18, characterised in that the microorganism is transformed with a recombinant plasmid according to any one of claims 9-13.

20. A biologically pure culture of Escherichia coli ELdTA2 (FERM BP-962), Escherichia coli ELdTD11 (FERM BP-963) or Escherichia coli ELdTD12 (FERM BP-964).

Claims for the following contracting states: Austria, Greece and Spain

1. A process for producing a human lymphotoxin polypeptide derivative (HLPD), which comprises culturing a transformed microorganism containing a recombinant plasmid containing a DNA fragment coding for the human lymphotoxin polypeptide derivative and capable of expressing HLPD in the culture medium, producing and accumulating the HLPD in the culture medium, and recovering the product HLPD.

2. A process according to claim 1, characterised in that the microorganism is a transformed Escherichia coli.

3. A process according to claim 1 or 2, characterised in that the microorganism is transformed with a plasmid containing an inserted DNA fragment which codes for a mature human lymphotoxin polypeptide having the amino acid sequence shown in Formula 1, but in which certain selected ones of the amino acid sequence are missing.

4. A process according to claim 1, 2 or 3, characterised in that the inserted DNA fragment codes for an amino acid sequence as specified, and in which at least one of the first twenty-two amino acids are missing.

5. A process according to claim 4, characterised in that the inserted DNA fragment codes for an amino acid sequence as specified, and in which either amino acids 1 to 22, 1 to 11 or 1 to 19 are missing.

6. A process according to any one of claims 1-5, characterised in that the plasmid DNA has a tryptophan promoter and the said DNA fragment is incorporated into the plasmid DNA downstream from the tryptophan promoter.

7. A process according to any one of claims 1-6, characterised in that the recombinant plasmid is a plasmid herein identified as pLdTA2, pLdTD11 and pLdTD12.

8. A method according to any one of claims 1-7, characterised in that the microorganism is Escherichia coli ELdTA2 (FERM BP-962), Escherichia coli ELdTD11 (FERM BP-963) or Escherichia coli ELdTD12 (FERM BP-964).

9. A method of constructing a DNA coding for a human lymphotoxin polypeptide derivative of high LT activity, characterised in that the constructed DNA codes for the amino acid sequence set forth in Formula 1. running from amino acids Leu (No. 1) to Leu (No. 171), but with at least one of the first twenty-two amino acids missing.

10. A method according to claim 8, characterised in that the constructed DNA codes for an amino acid sequence as specified in which either amino acids 1 to 22, 1 to 11 or 1 to 19 are missing.

11. A method of forming a recombinant plasmid incorporating a DNA fragment coding for a human lymphotoxin polypeptide derivative, characterised by inserting into the plasmid a DNA fragment coding for the amino acid sequence from Leu (No. 1) to Leu (No. 171) as shown in Formula 1, but modified in that certain of the amino acids are missing or are removed.

12. A method according to claim 11, characterised in that the inserted DNA fragment codes for the amino acid sequence shown in Formula 1 but in which amino acids 1 to 22, 1 to 11 or 1 to 19 are missing.

13. A method according to claim 11 or 12, characterised in that the DNA fragment is inserted into the plasmid downstream of a tryptophan promoter.

14. A method of transforming a microorganism with a plasmid containing a DNA fragment coding for a human lymphotoxin polypeptide derivative of high LT activity, and capable of expressing that polypeptide derivative in a culture medium, characterised in that the microorganism is transformed with a plasmid containing a DNA fragment coding for the amino acid sequence from Leu (No. 1) to Leu (No. 171) as shown in Formula 1, but with selected ones of said amino acids missing.

15. A method according to claim 14, characterised in that the plasmid used contains a DNA fragment coding for said amino acid sequence, and in which one or more of amino acids 1 to 22 is missing.

16. A method according to claim 15, characterised in that said DNA fragment codes for said amino acid sequence, and in which amino acids 1 to 22, 1 to 11 or 1 to 19 are missing.

17. A method according to claim 14, 15 or 16, characterised in that the plasmid contains a tryptophan promoter upstream of said DNA fragment.

18. A method according to any one of claims 14-17, characterised in that the microorganism transformed is Escherichia coli.

Fig. 1

0252107

Fig. 2

0232107

27mer          Met Leu Pro Gly Val
5'-CGATAAGCTT ATG CTA CCA GGA GTA GG-3'
3'- TATTCGAA TAC GAT GGT CCT CAT CC-5'
          25mer

— phosphorylation

ligation with T4 DNA ligase

Fig. 3

0232107

Fig. 4

0252107

ligation with T4 DNA ligase

0232107

# FIG.5

FIG.6 (1)

0232107

# FIG.6 (2)

FIG.7

0232107

*
GGGGCTCCGCACAGCAGGTGAGGCTCTCCT

GCCCCATCTCCTTGGGCTGCCCGTGCTTCGGTGCTTTGGACTACCGCCCAGCAGTGTCCTGCCCTCTGCCTGGGCCTCGGTCCCTCC

-34                -30
Met Thr Pro Pro Glu Arg Leu Phe Leu
ATG ACA CCA CCT GAA CGT CTC TCC CTC

TGCACCTGCTGCCTGGATCCCCGGCCTGCCTGGGCCTGGGCCTTGGTTCTCCCC

-20                                                         -10                              -1
Pro Arg Val Cys Gly Thr Thr Leu His Leu Leu Leu Leu Gly Leu Leu Leu Val Leu Leu Pro Gly Ala Gln Gly
CCA AGG GTG TGT GGC ACC ACC CTA CAC CTC CTC CTT CTG GGG CTG CTG CTG GTT CTG CTG CCT GGG GCC CAG GGG

1                                                  10                                             20
Leu Pro Gly Val Gly Leu Thr Pro Ser Ala Ala Gln Thr Ala Arg Gln His Pro Lys Met His Leu Ala His Ser
CTC CCT GGT GTT GGC CTC ACA CCT TCA GCT GCC CAG ACT GCC CGT CAG CAC CCC AAG ATG CAT CTT GCC CAC AGC

          30                                      40                                              50
Thr Leu Lys Pro Ala Ala His Leu Ile Gly Asp Pro Ser Lys Gln Asn Ser Leu Leu Trp Arg Ala Asn Thr Asp
ACC CTC AAA CCT GCT GCT CAC CTC ATT GGA GAC CCC AGC AAG CAG AAC TCA CTG CTC TGG AGA GCA AAC ACG GAC

                            60                                      70
Arg Ala Phe Leu Gln Asp Gly Phe Ser Leu Ser Asn Asn Ser Leu Leu Val Pro Thr Ser Gly Ile Tyr Phe Val
CGT GCC TTC CTC CAG GAT GGT TTC TCC TTG AGC AAC AAT TCT CTC CTG GTC CCC ACC AGT GGC ATC TAC TTC GTC

          80                                      90                                              100
Tyr Ser Gln Val Val Phe Ser Gly Lys Ala Tyr Ser Pro Lys Ala Thr Ser Ser Pro Leu Tyr Leu Ala His Glu
TAC TCC CAG GTG GTC TTC TCT GGG AAA GCC TAC TCT CCC AAG GCC ACC TCC TCC CCA CTC TAC CTG GCC CAT GAG

                            110                                     120
Val Gln Leu Phe Ser Ser Gln Tyr Pro Phe His Val Pro Leu Leu Ser Ser Gln Lys Met Val Tyr Pro Gly Leu
GTC CAG CTC TTC TCC TCC CAG TAC CCC TTC CAT GTG CCT CTC CTC AGC TCC CAG AAG ATG GTG TAT CCA GGG CTG

          130                                     140                                             150
Gln Glu Pro Trp Leu His Ser Met Tyr His Gly Ala Ala Phe Gln Leu Thr Gln Gly Asp Gln Leu Ser Thr His
CAG GAA CCC TGG CTG CAC TCG ATG TAC CAC GGG GCT GCG TTC CAG CTC ACC CAG GGA GAC CAG CTA TCC ACC CAC

                            160                                     170 171
Thr Asp Gly Ile Pro His Leu Val Leu Ser Pro Ser Thr Val Phe Phe Gly Ala Phe Ala Leu ***
ACA GAT GGC ATC CCC CAC CTA GTC CTC AGC CCT AGT ACT GTC TTC TTT GGA GCC TTC GCT CTG TAG   AACTTGGAA

AAATCCAGAAAGAAAAAATAATTGATTTCAAGACCTTCTCCCCATTCTGCCTCCATTCTGACCATTTCAGGGGTCGTCACCACCTCT

CCTTTGGCCATTCCAACAGCTCAAGTCTTCCCTGATCAAGTCACCGGAGCTTTCAAAGAAGGAATTCTAGGCATCCCAGGGGACCAC

ACCTCCCTGAACCATCCCTGATGTCTGTCTGGCTGAGGATTTCAAGCCTGCCTAGGAATTCCCAGCCCAAAGCTGTTGGTCTGTCCC

ACCAGCTAGGTGGGGCCTAGATCCACACACAGAGGAAGAGCAGGCACATGGAGGAGCTTGGGGGATGACTAGAGGCAGGGACGGGAC

TATTTATGAAGGCAAAAAAATTAAATTATTTATTTATGGAGGATGGAGAGAGGGGAATAATAGAAGAACATCCAAGGAGAAACAGAG

ACAGGCCCAAGAGATGAAGAGTGAGAGGGCATGCGCACAAGGCTGACCAAGAGAGAAAGAAGTAGGCATGAGGGATCACAGGGCCCC

AGAAGGCAGGGAAAGGCTCTGAAAGCCAGCTGCCGACCAGACCCCACACGGAGGCATCTGCACCCTCGATGAAGCCCAATAAACCTC

TTTTCTCTGAA

FIG.8

0232107

# FIG.9

European Patent Office

Application number:

87300674.6

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

Accession numbers of the deposits:

FERM BP. 981
FERM BP 963 - 964 - 962